# EUROPEAN PATENT SPECIFICATION

(11) **EP 1 803 463 B1**
(45) Date of publication and mention of the grant of the patent: **22.04.2009**
(21) Application number: 06398017.1
(22) Date of filing: 02.11.2006
(51) Int. Cl.: A61K 36/38, A61P 31/06

(54) **Extracts from hypericum L. species useful for the treatment of persistent tuberculosis**
Extrakte von Hypericum L. Arten zur Verwendung für die Behandlung von persistierender Tuberkulose
Extraits d'espèces d'hypericum L. destinés à être utilisés dans le traitement de la tuberculose persistante

(30) Priority: 02.11.2005 PT 10337705
(43) Date of publication of application: 04.07.2007
(73) Proprietor: Instituto Nacional de Engenharia, Tecnologia e Inovação, I.P., 1649-038 Lisboa (PT)
(72) Inventor: Duarte Dias Mendes Nogueira, Maria Teresa, 1200-355 Lisboa (PT); Gonçalves Da Costa, Maria do Céu, 1750-031 Lisboa (PT); Martins Moiteiro, Cristina Maria, 1600-427 Lisboa (PT); Preto Xavier Lobo Moutinho Medeiros, Maria Augusta, 2765-327 Estoril (PT); Vidal de Oliveira Baptista Marcelo Curto, Maria João, 2780-179 Oeiras (PT); Luna-Herrera, Julieta, 07920 México DF (MX); Reyes Rodriguez, Erika del Carmen, Atizapan Estado de México CP 52998 (MX)
(74) Representative: Pereira da Cruz, Joao

(56) References cited:
- WO-A-99/64027
- TOSUN F ET AL: "Antimycobacterial screening of some Turkish plants" JOURNAL OF ETHNOPHARMACOLOGY, ELSEVIER SCIENTIFIC PUBLISHERS LTD, IE, vol. 95, no. 2-3, December 2004 (2004-12), pages 273-275, XP004990577 ISSN: 0378-8741
- AVATO PINAROSA ET AL: "Determination of major constituents in St. John's Wort under different extraction conditions" February 2004 (2004-02), PHARMACEUTICAL BIOLOGY, VOL. 42, NR. 1, PAGE(S) 83-89 , XP009081733 ISSN: 1388-0209 * page 84 *
- RABANAL R M ET AL: "Analgesic and topical anti-inflammatory activity of Hypericum canariense L. and Hypericum glandulosum Ait" JOURNAL OF ETHNOPHARMACOLOGY, ELSEVIER SCIENTIFIC PUBLISHERS LTD, IE, vol. 96, no. 3, 15 January 2005 (2005-01-15), pages 591-596, XP004689963 ISSN: 0378-8741
- SMELCEROVIC A ET AL: "The separation of hypericine and pseudohypericine from Hypericum perforatum L." 2002, PHARMAZIE 2002 GERMANY, VOL. 57, NR. 3, PAGE(S) 178-180 , XP001245652 ISSN: 0031-7144 * page 179, right-hand column *
- RIVERA D ET AL: "THE ETHNOPHARMACOLOGY OF MADEIRA AND PORTO SANTO ISLANDS, A REVIEW" JOURNAL OF ETHNOPHARMACOLOGY, ELSEVIER SCIENTIFIC PUBLISHERS LTD, IE, vol. 46, no. 2, 1995, pages 73-93, XP001104547 ISSN: 0378-8741
- GUSEINOVA V E ET AL: "Examining the antimicrobial properties of medicinal plant species" 1992, FARMATSIYA (MOSCOW), VOL. 41, NR. 4, PAGE(S) 21-24 , XP009081710 ISSN: 0367-3014 abstract

## Description

### BACKGROUND OF THE INVENTION

Tuberculosis is a chronic infectious disease caused by *Mycobacterium tuberculosis* and is the leading killer in the world. Nowadays it is estimated that one third of the world's population is infected with *M. tuberculosis.* The World Health Organization (WHO) provide that in the next 20 years one billion people will be infected, 200 million will became sick and 35 million will die (Dias, J.C.; Rebelo, M.M.; Alves, C.N., Journal of Molecular Structure (Theochem) 676, 2004, 83-87).

In 1993 the WHO stated that tuberculosis was a global emergency disease. Although a vaccine (BCG) and effective chemotherapy against tuberculosis were available 50 years ago, strains that are resistant to all major anti-tuberculosis drugs have emerged (WHO Report 2005, Global Tuberculosis Control). Also the HIV/AIDS epidemic has resulted in the appearance of additional drug resistant isolates (El Sayed, K.A.; Bartyzel, P.; Shen, X.; Perry, T.L.; Zjawiony, J.K.; Hamann, M.T., Tetrahedron, 56, 2000, 949-953) what demands new treatments for this mortal disease (Okunade, A.L.; Elvin-Lewis, M.P.F.; Lewis, W.H., Phytochemistry, 65, 2004, 1017-1032).

This underscores the urgent need for the development of new structural classes of anti-tuberculosis agents to replace and/or to supplement the current drug regimens (Jain, R.; Vaitilingam, B.; Nayyar, A.; Palde. P.B., Bioorganic & Medicinal Chemistry Letters, 13, 2003, 1051-1054).

The increasing of this disease shows that the scientific community has slowly answer to the growing evidence of rising incidence of tuberculosis. Thus, the use of active extracts, according to a fast method preparation, may constitute one way of overcoming the long time taken from the bioactivity evaluation to the identification of the responsible molecule.

*Hypericum perforatum* L. is a species of *Hypericum* L. genus used as medicinal plant for over 2000 years. Pharmacological activities such as antidepressant, antiviral (anti-retroviral, including anti-HIV effects) and antibacterial have been referred in more than 800 publications.

In the search for antituberculosis compounds the authors paid special attention to the preliminary results described for the extracts of *H. calycinum (*Gottshall, R.Y., Lucas, E. H. , Lickfeldt, A. , Roberts, J.M., J. Clin. Invest. 28, 1949, 920-923*),* which presented positive qualitative activity for *M. tuberculosis* and negative activity for *Staphylococcus aureus.* The authors surprisingly obtained the opposite results (MIC 25 µg/mL) for *Staphylococcus aureus* (T. Nogueira, M.C. Costa, C. Moiteiro, A. Medeiros, S. Feio, M.J. Marcelo-Curto, A. Arellanes, E. Reye and J. Luna-Herrera, New data on biological activity in *Hypericum* L. taxa from Portugal, paper in preparation). The positive activity of *H. perforatum* against *M. tuberculosis,* by disk diffusion method at a concentration of 100 mg extract/disk (McCutcheon, A.R., Stokes, R.W., Thorson, L.M., Ellis, S.M., Hancock, R.E.W., Towers, G.H.N., Int. J. Pharmacog., 35, 1997, 77-83) was also surprisingly contradictory with the authors results (MIC > 200 µg/mL). More recently, it was referred the anti-*M. tuberculosis* H₃₇Ra activity of ethanol extract of *H. triquetrifolium* Turra, with a Minimal inhibitory concentration (MIC) of 100 µg/mL (Tosun, F., Akyüz Kizilay, Ç., Sener, B., Vural, M., Palittapongarnpim, P., J. of Ethnopharmacology 95, 2004, 273-275).

Some QSAR studies referred quinones (Cellai *et al.,* 1980-2000) and biflavonoids (Alves et al., J. Mol. Structure, 676, 2004, 83-87) as active substances against tuberculosis. The authors have already published a first analytical study on the majority of portuguese *Hypericum* species (A. Farinha, J. M. Martins, T. Nogueira, R. Tavares e A.F. Duarte, Natural Products in the New Millennium: Prospects and Industrial Application, 2002, 125-134, Kluwer Academic Publishers) and the absence of hypericin was confirmed in some *Hypericum* species (*H. androsaemum, H. calycinum, H. elodes, H. hircinum* subsp. *majus* and *H. pulchrum*). The higher content of flavonoids were obtained for *H. androsaemum, H.* calycinum, *H. humifusum, H. linarifolium, H. montanum, H. perforatum, H. pulchrum, H. perfoliatum, H. tomentosum* and *H. undulatum* (≈1%), being the last one the species where all the compounds analyzed were present (rutin, hyperosid, kaempferol, amentoflavon, isoquercetrin, quercitrin, quercetin, cafeic and chlorogenic acids).

The present invention rests on the surprising finding that there are active extracts of *Hypericum* species, never tested before, with low MIC values (12.5 to 200 µg/mL) to resistant strains and clinical isolates that may constitute a source of new leads towards the discovery of new potential candidates for the treatment of tuberculosis.

### DETAILED DESCRIPTION OF THE INVENTION

A first object of the invention is the preparation of new extracts of *Hypericum* spp. with high anti-tuberculosis activity. These extracts are obtained by powdering the aerial parts of *H. humifusum* or *H. calycinum* or *H. elodes* or *H. hircinum* subsp. *majus* or *H. grandifolium* or *H. foliosum,* followed by extraction of the plant material in hydroxylated solvent or in mixtures with water on the proportion of 1:0.5 or 1:1 and maceration for 12 to 36 hours, in order to obtain a first extract, followed by sonication for 10 to 50 minutes, especially about 30 minutes, filtration and evaporation to dryness under low pressure and at below 3.5 °C in order to obtain an active concentrate.

In a preferred embodiment of the invention, the method for the preparation of *Hypericum* extracts consists on the proportion of plant material : solvent of 0.5 g to 100 mL, respectively, the concentrate being diluted with water or mixtures of hydroxylated solvent - water, with maceration time of 24 hours, in the absence of light and followed by the screening of biological activity.

In preferred embodiments of the invention, the concentrated extracts of *Hypericum* spp. obtained as described above are characterized by antimycobacterial activity against *M. tuberculosis* H₃₇Rv, MIC values from 25 to 200 µg/mL, determined by a microcolorimetric assay with Alamar Blue dye (MABA) (Jimenez-Arellanes, A., Meckes, M., Ramirez, R., Torres, J. and Luna-Herrera, J., 2003, Phytother. Res., 17: 903-908).

In preferred embodiments of the invention, the concentrated extracts of *Hypericum* spp. obtained according to the invention are also characterized by MIC values from 25 to 100 µg/mL of activity of rifampicin-resistant strain against *M. tuberculosis.*

Moreover, in another preferred embodiment of the invention, the concentrated extracts of *Hypericum* spp. obtained according to the invention are also characterized by MIC values from 25 to 100 µg/mL of activity of streptomycin-resistant strain against *M. tuberculosis* and characterized by MIC values from 50 to 100 µg/mL of ethambutol-resistant strain activity against *M. tuberculosis.*

In addition to another preferred embodiment of the invention, the concentrated extracts of *Hypericum* spp. obtained according to the invention are also characterized by MIC values from 25 to 100 µg/mL of activity of isoniazid-resistant strain against *M. tuberculosis* and MIC values from 12.5 to 100 µg/mL of clinical isolates activity against *M. tuberculosis.*

In another more preferred embodiment, the concentrated extracts obtained according to the invention are characterized by the presence of hyperforin, hypericin and pseudohypericin quantified by an analytical method of high performance liquid chromatography, which isolated activities have MIC ≥ 200 µg/mL and characterized for having low MICs (≤ 200 µg/mL) in the absence of hypericin.

The concentrated extracts of *Hypericum* spp. obtained as described above are useful in the treatment of persistent tuberculosis.

A second object of the invention is a method for the preparation of concentrated extracts of *Hypericum* spp. obtained as described above.

A third object of the invention relates to the use of concentrated extracts of *Hypericum* spp. obtained as described above, in pharmaceutical preparations for the treatment of persistent tuberculosis.

A fourth object of the invention is a pharmaceutical composition containing a concentrated extract of *Hypericum* spp., obtained as described above, with an excipient or a vehicle pharmaceutically acceptable.

A fifth object of the invention is a method for the treatment of persistent tuberculosis using concentrated extracts of *Hypericum* spp. obtained as described above.

### EXPERIMENTAL

### Equipment and reagents

All the solvents used were analytical grade and are mainly hydroxylated.

Reference compounds of hypericin, pseudohypericin and hyperforin were purchased from Chromadex, Inc. USA.

A ventilated oven (Cassel, with air circulation to the exterior), grinder (Tecator, Cemotec, 1090), ultrasounds, rotative evaporator (Rotavapor, Büchi) were used.

96-well sterile microplates (Nunc), 7H9 broth, incubator, Alamar Blue solution (Trek diagnostic, Westlake, Ohio), sterile Tween solution were also used.

### EXAMPLES

### Preparation of dry extracts of H. humifusum, H. calycinum, H. elodes, H. hircinum subsp. majus, H. grandifolium and H. foliosum.

The aerial part of each plant species was collected and dried in a ventilated oven at 35°C during 10 days and mechanically grinded. About 500 mg of powdered plant material was extracted with 100 mL of an hydroxylated solvent for 24 h by maceration, at a temperature of 25°C, followed by sonication during 30 min. Extracts were filtered for the residues removal and evaporated to dryness under low pressure and at a temperature lower than 35°C, in order to obtain concentrates.

### Antimycrobial screening for the revaluation of extracts susceptibility to M. tuberculosis strains by a microcolorimetric assay with Alamar Blue

The antimycobacterial assays were performed using *Mycobacterium* species obtained from the American Type Culture Collection (ATCC, Rockville, MD): *Mycobacterium tuberculosis* H₃₇Rv (ATCC 27294), H₃₇Rv isoniazid-resistant (ATCC 35822), H₃₇Rv rifampicin-resistant (ATCC 35838), H₃₇Rv ethambutol-resistant (ATCC 35837). Four drug-resistant pulmonar isolates of *M. tuberculosis* were obtained from patients at different hospitals in Mexico. The selection of these drug-resistant isolates was based on their drug sensitivity pattern to the antimycobacterial drugs isoniazid, rifampicin, rifabutin, ethambutol, ofloxacin, clarithromycin, streptomycin and ethionamide.

This test was performed in 96-well sterile microplates (Nunc) in duplicate for each extract. To each well 100 µL of 7H9 broth, 100 µL of each extract solutions with 6 concentrations in the range 100-3.25 µg/mL and 100 µL of each suspension of 4 strains of *M. tuberculosis* and 4 isolates of drug resistant *M. tuberculosis* obtained from patients of different hospitals were added.

Similar assays were performed by adding the bacterial suspensions of the different strains of *M. tuberculosis* to the drug-free control wells which included 100% of the bacterial inoculums and controls with 1% of the bacterial inoculums which present a colour or fluorescence unity for the measurement of MIC.

The plates were incubated at 37°C. After 5 days of incubation for *M. tuberculosis,* 20 µL of Alamar blue solution and 12 µL of sterile 10% Tween 80 were added to the wells. The colour analysis was done by comparison with control plates and determined the minimal inhibitory concentration as the lowest extract concentration that prevented a colour change or the one that presents a fluorimetric measure lesser than the one of the control with 1% of the inoculums and represents an inhibition of 99% of bacterial culture.

The results obtained are presented in the following table.

**TABLE**

| **Minimal inhibitory concentrations (µg mL⁻¹) of extracts of *Hypericum* spp. and reference compounds against *M. tuberculosis* H37Rv, resistant strains and clinical isolates** | | | | | | | | | |
|---|---|---|---|---|---|---|---|---|---|
| | **MIC (µg mL⁻¹)** | | | | | | | | |
| | | Drug-resistant strains | | | | Drug-resistant clinical isolates | | | |
| ***Hypericum* spp.** | H37Rv | H37Rv INH-R | H37Rv ETAM-R | H37Rv SM-R | H37Rv RIF-R | Is.cl. SIN-3 | Is.cl. SIN-4 | Is.cl. HGB | Is.cl. MMDO |
| *H. calycinum* | 100 | 100 | >100 | 100 | 100 | 100 | >100 | 100 | 100 |
| *H. elodes* | 50 | 50 | 50 | 25 | 50 | 50 | 50 | 50 | 50 |
| *H. humifusum* | 200 | 100 | 50 | 50 | 50 | >100 | >100 | 100 | >100 |
| *H. hircinum* ssp. *majus* | 25 | 25 | 100 | 50 | 25 | 12.5 | 100 | 50 | 50 |
| *H. foliosum* | 200 | 50 | 50 | 100 | 100 | n.t. | n.t. | n.t. | 100 |
| *H. grandifolium* | 200 | 50 | 50 | 100 | 100 | n.t. | n.t. | n.t. | 100 |

| **Compounds** | | | | | | | | | |
|---|---|---|---|---|---|---|---|---|---|
| Hypericin | >200 | >50 | >50 | n.t. | n.t. | n.t. | n.t. | n.t. | >50 |
| Pseudohypericin | >200 | >50 | >50 | n.t. | n.t. | n.t. | n.t. | n.t. | >50 |
| Hyperforin | 200 | >50 | 50 | >50 | >50 | n.t. | n.t. | n.t. | >50 |

| | | | | | | | | | |
|---|---|---|---|---|---|---|---|---|---|
| n.t. - not tested; MIC ≤ 200 µg mL⁻¹ - active. | | | | | | | | | |

### Mycobacterium tuberculosis strains and clinical isolates:

H37Rv - reference strain, H37Rv INH-R - isoniazid - resistant strain, H37Rv ETAM-R - ethambutol - resistant strain, H37Rv SM-R - streptomycin - resistant strain, H37Rv RIF-R - rifampicin - resistant strain, Is.cl.SIN-3 - resistant to streptomycin, isoniazid, rifampicin, rifabutin, ethambutol, clarithromycin and ofloxacin, Is.cl.SIN-4 - resistant to streptomycin, isoniazid, rifampicin, rifabutin, ethambutol, ethionamide and ofloxacin, Is.cl.HG8 - resistant to ethambutol, clarithromycin and ethionamide, Is.cl.MMDO - resistant to isoniazid and ethambutol.

## Claims

1. Extracts of *Hypericum* spp. for use in the treatment of persistent tuberculosis, **characterized in that** they are prepared from the aerial part of the species *H. humifusum* or *H. calycinum* or *H. elodes* or *H. hircinum* subsp. *majus* or *H. grandifolium* or *H. foliosum* by grinding the plant material (0,5 g), followed by extraction with 100 mL of an hydroxylated solvent or with mixtures 1:0,5 or 1:1 of the hydroxylated solvent with water and maceration for 12 to 36 hours to give a first extract that is sonicated for 10-50 minutes, filtered, evaporated to dryness under low pressure and at a temperature lower than 35 °C, in order to obtain a concentrate.

2. Extracts of *Hypericum* spp. for use in the treatment of persistent tuberculosis, according to claim 1, **characterized in that** the dilution of the concentrate in water or in mixtures of water-hydroxylated solvent are carried out.

3. Extracts of *Hypericum* spp. for use in the treatment of persistent tuberculosis, according to any one of the previous claims, **characterized in that** the maceration time is 24 hours in the absence of light.

4. Extracts of *Hypericum* spp. for use in the treatment of persistent tuberculosis, according to any one of the previous claims, **characterized in that** the time of sonication of the extracted solution is 30 minutes.

5. Extracts of *Hypericum* spp. for use in the treatment of persistent tuberculosis, according to any one of the previous claims, **characterized in that** they exhibit Minimal Inhibitory Concentrations (MICs) from 25 to 200 µg/mL for the *Mycobacterium* tuberculosis H₃₇Rv using the microcolorimetric Alamar Blue Assay (MABA).

6. Extracts of *Hypericum* spp. for use in the treatment of persistent tuberculosis, according to any one of the previous claims, **characterized in that** they exhibit MICs from 25 to 100 µg/mL for the rifampicin-resistant strain of *Mycobacterium tuberculosis.*

7. Extracts of *Hypericum* spp. for use in the treatment of persistent tuberculosis, according to any one of the claims 1 to 5, **characterized in that** they exhibit MICs from 25 to 100 µg/mL for the streptomycin-resistant strain of *Mycobacterium tuberculosis*.

8. Extracts of *Hypericum* spp. for use in the treatment of persistent tuberculosis, according to any one of the claims 1 to 5, **characterized in that** they exhibit MICs from 50 to 100 µg/mL for the ethambutol-resistant strain of *Mycobacterium tuberculosis*.

9. Extracts of *Hypericum* spp. for use in the treatment of persistent tuberculosis, according to claims 1 to 5, **characterized in that** they exhibit MICs from 25 to 100 µg/mL for the isoniazid-resistant strain of *Mycobacterium tuberculosis.*

10. Extracts of *Hypericum* spp. for use in the treatment of persistent tuberculosis, according to claims 1 to 5, **characterized in that** they exhibit MICs from 12.5 to 100 µg/mL for the strain of *Mycobacterium tuberculosis* from clinical isolates.

11. Extracts of *Hypericum* spp. for use in the treatment of persistent tuberculosis, according to previous claims, **characterized in that** they comprise hyperforin, hypericin and pseudohypericin in amounts quantified by an analytical method of high performance liquid chromatography, whose isolated activities against *M.* tuberculosis H37RV have MICs ≥ 200 µg/mL, against drug-resistant strains have MICs ≥ 50 µg/mL and against isoniazid and ethambutol resistant clinical isolates have MICs > 50 µg/mL.

12. Extracts of *Hypericum* spp. for use in the treatment of persistent tuberculosis, according to claims 1 to 10, **characterized in that** they have low MICs (≤ 200 µg/mL) in the absence of hypericin.

13. Extracts of *Hypericum* spp. for use in the treatment of persistent tuberculosis, according to claim 12, **characterized in that** they have MICs of 100 µg/mL for *H. calycinum*, 50 µg/mL for *H. elodes,* 25 µg/mL for *H. hircinum* subsp- *majus*,200 µg/mL for *H. foliosum* and 200 for µg/mL *H. grandifolium*,against M. tuberculosis H37Rv.

14. Extracts of *Hypericum* spp. for use in the treatment of persistent tuberculosis, according to claims 1 to 11, **characterized in that** they have MICs of 200 µg/mL for *H. humifusum* against *M. tuberculosis* H37Rv in the presence of hypericin.

15. Extracts of *Hypericum* spp., according to any one of the previous claims, for use in the treatment of persistent tuberculosis.

16. Method of preparation of extracts of *Hypericum* spp. for use in the treatment of persistent tuberculosis, **characterized in that** they are prepared from the aerial part of the species *H. humifusum* or *H. calycinum* or *H. elodes* or *H. hircinum* subsp. *majus* or *H. grandifolium* or *H. foliosum* by the following steps:
a) grinding the plant material;
b) extraction with an hydroxylated solvent or with mixtures 1:0,5 or 1:1 of the hydroxylated solvent with water;
c) maceration for 12 to 36 hours to give a first extract;
d) sonication for 10-50 minutes;
e) filtration; and
f) evaporation to dryness under low pressure and at a temperature lower than 35 °C, in order to obtain a concentrate; and, optionally,
g) dilution of the concentrate in water or in mixtures of water-hydroxylated solvent.

17. Use of extracts of *Hypericum* spp., according to any of the claims 1 to 15, for the manufacture of pharmaceutical preparations for the treatment of persistent tuberculosis.

18. Pharmaceutical preparation for use in the treatment of persistent tuberculosis, **characterized in that** it consists of one of the extracts of *Hypericum* spp., according to any one of claims 1 to 15, and a pharmaceutically acceptable excipient or vesicle.

## Patentansprüche

1. Extrakte aus *Hypericum* spp. zur Verwendung für die Behandlung von persistierender Tuberkulose **dadurch gekennzeichnet, daß** sie aus an der Luft wachsenden Teilen der Spezien *H. humifusum* oder *H. calcynum* oder *H. elodes* oder *H. hircinum* subsp. *majus* oder H. *grandifolium* oder *H. foliosum* hergestellt warden mittels Zermahlen des Pflanzenmaterials (0.5 g), und anschliessender Extraktion mit 100 mL eines hydroxylierten Lösungsmittels oder mit Mischungen von 1:0.5 oder 1:1 dieses hydroxylierten Lösungsmittels mit Wasser, sowie Einweichen während 12 bis 36 Stunden um den ersten Extrakt zu erhalten, der 10-50 Minuten lang mit Ultraschall behandelt wird, danach filtriert wird und dann bis zur vollkommenen Trockne bei niedrigem Druck und bei einer Temperatur unter 35°C verdamft Wird, um ein Konzentrat zu erhalten.

2. Extrakte aus Hypericum spp. zur Verwendung für die Behandlung von persistierende Tuberkulose nach Anspruch 1, **dadurch gekennzeichnet, daß** die Auflösung des Konzentrats in Wasser oder in Mischungen von Wasserhydroxylierten Lösungsmitteln durchgeführt wird.

3. Extrakte aus Hypericum spp. zur Verwendung für die Behandlung von persistierende Tuberkulose nach einem der vorgennanten Ansprüchen**, dadurch gekennzeichnet, daß** die Einweichzeit ohne Lichteinfluß, d.h. bei völliger Dunkelheit während 24 Stunden durchgefürt wird.

4. Extrakte aus Hypericum spp. zur Verwendung für die Behandlung von persistierende Tuberkulose nach einem der vorgennanten Ansprüchen, **dadurch gekennzeichnet, daß** die Zeit der Ultraschallbehandlung der erhaltenen Lösung 30 Minuten ist.

5. Extrakte aus *Hypericum* spp. zur Verwendung für die Behandlung von persistierende Tuberkulose nach einem der vorgennanten Ansprüchen, **dadurch gekennzeichnet, daß** die minimale Hemmungskonzentration (MICs) 25 bis 200 µg/mL für die *Mycobacterium tuberculosis* H37Rv beträgt mit Verwendung von mikrocolorimetrische Alamar Blue Assay (MABA).

6. Extrakte aus *Hypericum* spp. zur Verwendung für die Behandlung von persistierende Tuberkulose nach einem der vorgennanten Ansprüchen, **dadurch gekennzeichnet, daß** sie für den Rifampicin-resistenten Stamm der *Mycobacterium tuberculosis* MICS von 25 bis 100 µg/mL betragen.

7. Extrakte aus *Hypericum* spp. zur Verwendung für die Behandlung von persistierende Tuberkulose nach einem der vorgennanten Ansprüchen 1 bis 5, **dadurch gekennzeichnet, daß** sie für den Streptomycin-resistenten Stamm der *Mycobacterium* tuberculosis MICs von 25 bis 100 µg/mL betragen.

8. Extrakte aus *Hypericum* spp. zur Verwendung für die Behandlung von persistierende Tuberkulose nach einem der vorgennanten Ansprüchen 1 bis 5, **dadurch gekennzeichnet, daß** sie für den Ethambutol-resistenten Stamm der *Mycobacterium tuberculosis* MICs von 50 bis 100 µg/mL für den Ethambutol-resistenten betragen.

9. Extrakte aus *Hypericum* spp. zur Verwendung für die Behandlung von persistierende Tuberkulose nach den Ansprüchen 1 bis 5, **dadurch gekennzeichnet, daß** sie für den Isoniazid-resistenten Stamm der *Mycobacterium tuberculosis* MICs von 25 bis 100 µg/mL betragen.

10. Extrakte aus *Hypericum* spp. zur Verwendung für die Behandlung von persistierende Tuberkulose nach den Ansprüchen 1 bis 5, **dadurch gekennzeichnet, daß** sie für den Stamm der *Mycobacterium tuberculosis* aus klinischen Isolaten MICs von 12.5 bis 100 µg/mL betragen.

11. Extrakte aus *Hypericum* spp. zur Verwendung für die Behandlung von persistierende Tuberkulose nach den vorhergehenden Ansprüchen, **dadurch gekennzeichnet, daß** sie Hyperforin, Hypericin und Pseudohypericin umfassen, zwar in Mengen durch eine analytische Methode, High Performance Liquid Chromatography (HPLC) quantifiziert wird, deren isolierte Aktivitäten gegen *M. tuberculosis* H37RV MICs ≥ 200 µg/mL, gegen Drogen-resistente Stammen MICs ≥ 50 µg/mL und gegen Isoniazid und Ethambutol resistente klinische Isolate MICs > 50 µg/mL ergeben.

12. Extrakte aus *Hypericum* spp. zur Verwendung für die Behandlung von persistierende Tuberkulose nach Ansprüchen 1 bis 10, **dadurch gekennzeichnet, daß** sie bei Abwesenheit von Hypericin niedrige MICs (≤ 200 µg/mL) ergeben.

13. Extrakte aus *Hypericum* spp. zur Verwendung für die Behandlung von persistierende Tuberkulose nach Ansprüch 12, **dadurch gekennzeichnet, daß** sie MICs von 100 µg/mL für *H. calcynum,* 50 µg/mL für *H. elodes,* 25 µg/mL für *H. hircinum* subsp. majus, 200 µg/mL für *H.foliosum* und 200 µg/mL für H. grandifolium gegen die *M. tuberculosis* H37Rv ergeben.

14. Extrakte aus *Hypericum* spp. zur Verwendung für die Behandlung von persistierende Tuberkulose nach Ansprüchen 1 bis 11, **dadurch gekennzeichnet, daß** sie MICs von 200 µg/mL für *H. humifusum* gegen die *M. tuberculosis* H37Rv bei Anwesenheit von Hypericin ergeben.

15. Extrakte aus *Hypericum* spp. nach eine der vorgennanten Ansprüchen für die Behandlung von persistierender Tuberkulose.

16. Methode für die Herstellung von Extrakten des *Hypericum* spp. zur Verwendung für die Behandlung von persistierende Tuberkulose, **dadurch gekennzeichnet, daß** die an der Luft wachsenden Teile der Spezien *H.* humifusum oder *H. calcynum* oder *H. elodes* oder *H. hircinum* subsp. *majus* oder *H. grandifolium* oder *H. foliosum* folgendermaßen erfolgt:
a) Zermahlen des Pflanzenmaterials;
b) Extraktion durch hydroxyliertes Lösungsmittel oder mit Mischungen von 1:0.5 oder 1:1 des hydroxylierten Lösungsmittels mit Wasser;
c) Einweichen während 12 bis 36 Stunden um den ersten Extrakt zu erhalten;
d) Ultraschall von 10 bis 50 Minuten;
e) Filtration; und
f) Verdamfung bis zu vollkommene Trocknung bei niedrigem Druck und bei einer Temperatur unter 35°C, um einen Konzentrat zu erhalten; und gegebenenfalls
g) Auflösung des Konzentrates in Wasser oder in Mischungen aus Wasser-hydroxylerten Lösungsmitteln.

17. Verwendung von Extrakte des *Hypericum* spp., nach den oben gennanten Ansprüche 1 bis 15, für die Herstellung von pharmazeutischen Zubereitungen für die Behandlung von persistierende Tuberkulose.

18. Pharmazeutische Zubereitung für Anwendung zur Behandlung von persistierende Tuberkulose, **dadurch gekennzeichnet, daß** es sich um einem der Extrakte des *Hypericum* spp. handelt, nach einem der Ansprüche 1 bis 15, und ein pharmazeutisch verträgliches Zusatzstoffes oder Träger.

## Revendications

1. Extraits d'espèces d'*Hypericum* pour utilisation dans le traitement de la tuberculose persistante, **caractérisés en ce qu'**ils sont preparés à partir de la partie aérienne des espèces *H. humifusum* ou *H. calycinum* ou *H. elodes* ou *H. hircinum* subsp. *majus* ou *H. grandifolium* ou *H. foliosum* par trituration du matériel végétal (0,5 g), suivie par extraction avec 100 mL d'un solvent hydroxylé ou avec des mélanges 1:0,5 ou 1:1 du solvent hydroxylé avec de l'eau et macération pendant 12 à 36 heures pour donner um premier extrait qui est soniqué pendant 10-50 minutes, filtré, évaporé jusqu'au séchage sécheresse à basse pression et à une temperature inférieure à 35°C, pour obtenir un concentré.

2. Extraits d'espèces d'*Hypericum* pour utilisation dans le traitement de la tuberculose persistante suivant la revendication 1, **caractérisés en ce qu'**on fait la dilution du concentré dans l'eau ou dans des mélanges d'eau-solvent hydroxylé.

3. Extraits d'espèces d'*Hypericum* pour utilisation dans le traitement de la tuberculose persistante suivant l'une quelconque des revendications antérieures, **caractérisés en ce que** le temps de macération est de 24 heures dans l'absence de lumière.

4. Extraits d'espèces d'*Hypericum* pour utilisation dans le traitement de la tuberculose persistante suivant l'une quelconque des revendications antérieures, **caractérisés en ce que** le temps de sonication de la solution extraite est de 30 minutes.

5. Extraits d'espèces d'*Hypericum* pour utilisation dans le traitement de la tuberculose persistante suivant l'une quelconque des revendications antérieures, **caractérisés en ce qu'**ils présentent des concentrations inhibitoires minimales (MICs) de 25 à 200 µg/mL pour le *Mycobaterium tuberculosis* H37Rv utilisant l'Essai du Bleu d'Alamar n-micro-calorimétrique(MABA).

6. Extraits d'espèces d'*Hypericum* pour utilisation dans le traitement de la tuberculose persistante suivant l'une quelconque des revendications antérieures, **caractérisés en ce qu'**ils présentent des MICs de 25 à 100 µg/mL pour la souche de *Mycobaterium tuberculosis* résistante à la rifampicine.

7. Extraits d'espèces d'*Hypericum* pour utilisation dans le traitement de la tuberculose persistante suivant l'une quelconque des revendications 1 à 5, **caractérisés en ce qu'**ils présentent des MICs de 25 à 100 µg/mL pour la souche de *Mycobaterium tuberculosis* résistante à la streptomycine.

8. Extraits d'espèces d'*Hypericum* pour utilisation dans le traitement de la tuberculose persistante suivant l'une quelconque des revendications 1 à 5, **caractérisés en ce qu'**ils présentent des MICs de 50 à 100 µg/mL pour la souche de *Mycobaterium tuberculosis* résistante à l'éthambutol.

9. Extraits d'espèces d'*Hypericum* pour utilisation dans le traitement de la tuberculose persistante suivant les revendications 1 à 5, **caractérisés en ce qu'**ils présentent des MICs de 25 à 100 µg/mL pour la souche de *Mycobaterium tuberculosis* résistante à l'isoniazide.

10. Extraits d'espèces d'*Hypericum* pour utilisation dans le traitement de la tuberculose persistante suivant les revendications 1 à 5, **caractérisés en ce qu'**ils présentent des MICs de 12,5 à 100 µg/mL pour la souche de *Mycobaterium tuberculosis* d'isolés cliniques.

11. Extraits d'espèces d'*Hypericum* pour utilisation dans le traitement de la tuberculose persistante suivant les revendications antérieures, **caractérisés en ce qu'**ils comprennent l'hyperflorine, l'hypericine et la pseudo-hypericine en des quantités determinées par une méthode analytique de chromatographie liquide à haute performance, qui ont des activités isolées contre *M. tuberculosis* H37Rv avec des MICs ≥ 200 µg/mL, contre des souches résistantes aux médicaments avec des MICs ≥ 50 µg/mL et contre des isolés cliniques résistants à l'isoniazide et à l'éthambutol avec des MICAS > 50 µg/mL.

12. Extraits d'espèces d'*Hypericum* pour utilisation dans le traitement de la tuberculose persistante suivant les revendications 1 à 10, **caractérisés en ce qu'**ils présentent des MICs bas (≤ 200 µg/mL) dans l'absence de l'hypericine.

13. Extraits d'espèces d'*Hypericum* pour utilisation dans le traitement de la tuberculose persistante suivant la revendication 12, **caractérisés en ce qu'**ils présentent des MICs de 100 µg/mL pour *H. calycinum*, 50 µg/mL pour *H. elodes,* 25 µg/mL pour *H. hircinum* subsp. *majus*, 200 µg/mL pour *H. foliosum* et 200 µg/mL pour *H. grandofolium*, contre *M. tuberculosis* H37Rv.

14. Extraits d'espèces d'*Hypericum* pour utilisation dans le traitement de la tuberculose persistante suivant les revendications 1 à 11, **caractérisés en ce qu'**ils présentent des MICs de 200 µg/mL pour *H. humifusum* contre *M. tuberculosis* H37Rv dans la présence d'hypericine.

15. Extraits d'espèces d'*Hypericum* suivant l'une quelconque des revendications antérieures, pour utilisation dans le traitement de la tuberculose persistante.

16. Procedé de préparation d'extraits d'espèces d'*Hypericum* pour utilization dans le traitement de la tuberculose persistante, **caractérisé en ce qu'**ils sont preparés à partir de la partie aérienne des espèces *H. humifusum* ou *H. calycinum* ou *H. elodes* ou *H. hircinum* subsp. *majus* ou *H. grandifolium* ou *H. foliosum* par les pas suivants:
a) trituration du matériel végétal;
b) extraction avec un solvent hydroxylé ou avec des mélanges 1:0,5 ou 1:1 du solvent hydroxylé avec de l'eau;
c) macération pendant 12 à 26 heures pour donner un premier extrait;
d) sonication pendant 10-50 minutes;
e) filtration; et
f) évaporation jusqu'au séchage à basse pression et à une température inférieure à 35°C, pour obtenir um concentré; et, éventuellement,
g) dilution du concentré dans l'eau où dans des mélanges d'eau-solvent hydroxylé.

17. Utilisation d'extraits d'espèces d'*Hypericum* suivant l'une quelconque des revendications 1 à 15, pour la fabrication de préparations pharmaceutiques pour le traitement de la tuberculose persistante.

18. Préparation pharmaceutique pour utilisation dans le traitement de la tuberculose persistante, **caractérisée en ce qu'**elle consiste en un des extraits d'espèces d'*Hypericum*, suivant l'une quelconque des revendications 1 à 15, et un excipient ou véhicule pharmaceutiquement acceptable.
